# EUROPEAN PATENT APPLICATION

(11) **EP 4 349 974 A1**
(43) Date of publication of application: **10.04.2024**
(21) Application number: 22816270.7
(22) Date of filing: 25.03.2022
(51) Int. Cl.: C12N 7/00, C12N 15/113

(54) **METHOD FOR PREPARING REASSORTANT ROTAVIRUS**

(30) Priority: 31.05.2021 KR 20210070036
(71) Applicant: SK Bioscience Co., Ltd., Bundang-gu, Seongnam-si, Gyeonggi-do, 13494 (KR)
(72) Inventor: LEE, Jung-Eun, Seongnam-si, Gyeonggi-do 13494 (KR); KWON, Taewoo, Seongnam-si, Gyeonggi-do 13494 (KR)
(74) Representative: J A Kemp LLP
(86) International application number: PCT/KR2022/004185
(87) International publication number: WO 2022/255607

(57) **Abstract**

The present invention relates to a method for preparing a reassortant rotavirus, and provides a reverse genetics platform using an inhibitory RNA as selective pressure for the reassortant rotavirus. Through the method, reassortment probability dramatically increases so that a desired reassortant rotavirus can be prepared in a short time and at low cost.

## Description

### [Technical Field]

### Cross-reference to Related Application

The present application is based on, and claims priority from, Korean Patent Application No. 10-2021-0070036, filed on May 31, 2021, the disclosure of which is hereby incorporated by reference herein in its entirety.

### Technical Field

The present invention relates to a method for preparing a reassortant rotavirus, and provides a reverse genetics platform using an inhibitory RNA as selective pressure for the reassortant rotavirus.

### [Background Art]

Rotavirus is the single most important cause of gastroenteritis in infants and young children worldwide. Each year, approximately 100 million people under 5 years of age suffer from rotavirus gastroenteritis and approximately 600,000 people die in the world. Deaths occur mainly in developing countries, but prevalence rates are similar in developing and developed countries. Therefore, the development of a vaccine is of utmost importance in order to prevent rotavirus gastroenteritis and reduce the mortality rate due to rotavirus infection.

As rotavirus has a genome composed of 11 segments of double-stranded RNA (dsRNA), when multiple virus strains co-infect one cell, a reassortant virus in which RNA segments of different strains are mixed can be produced. For example, a reassortant virus, in which RNA segments of bovine rotavirus that are not virulent to humans and RNA segments of human rotavirus that causes rotavirus gastroenteritis in humans are mixed, can be produced. Among these bovine-human reassortant viruses, reassortant viruses, in which the segment encoding VP4, which shows virulence to humans, is composed of an RNA segment from bovine rotavirus, and the segment encoding VP7, which contains many neutralizing epitopes, is composed of an RNA segment from human rotavirus, are not virulent in humans and do not cause gastroenteritis, but they can induce the formation of neutralizing antibodies against human rotavirus and thus can be used as vaccines against human rotavirus. One such example is Rotateq^{®}, a pentavalent vaccine made by reassorting and attenuating rotaviruses isolated from bovin and human.

A common method of producing a reassortant virus is to simultaneously infect one cell line with two types of rotaviruses to obtain a reassortant virus, but reverse genetics can be applied to increase the probability of reassortment. The reverse genetics method involves transfecting an excessive amount of mRNA of a gene to be reassorted, or DNA that can be transcribed into the mRNA into a cell and simultaneously infecting the cell with helper rotavirus in order to induce reassortment of the transfected mRNA and the genes of the helper rotavirus.

However, even if the probability of reassortment is increased using reverse genetics, as reassortant viruses are very few in number and attenuated compared to wild-type (WT) viruses, unless selective pressure is applied, the ratio gradually decreases as passage progresses, making it very difficult to isolate and rescue the desired reassortant virus. Therefore, an appropriate selection process must be introduced to obtain a successful reassortant rotavirus.

The method currently used in the selection process is to use a monoclonal neutralizing antibody that specifically binds to and neutralizes the protein of the gene to be replaced (e.g., VP7 gene of bovine rotavirus), but does not bind to the protein of the replaced gene (e.g., VP7 gene of human rotavirus) (for example, US Patent No. 4,571,385). However, the method using the monoclonal neutralizing antibody requires a lot of time and cost to obtain the neutralizing antibody and has the problem of making it difficult to obtain a neutralizing antibody specific to the protein of a specific strain because rotavirus strains are composed of genes with similar sequences, functions, and structures. Therefore, there is a need for research and development on separate technology that can apply selective pressure.

### [Disclosure]

### [Technical Problem]

The present invention relates to a method for preparing a reassortant rotavirus, and provides a reverse genetics platform using an inhibitory RNA as selective pressure for the reassortant rotavirus.

### [Technical Solution]

An embodiment described herein provides a method for producing a reassortant rotavirus comprising: providing a cell line stably expressing an inhibitory RNA against a specific gene segment among 11 gene segments of a first rotavirus; introducing mRNA of a gene segment of a second rotavirus corresponding to said specific gene segment of the first rotavirus, or DNA or cDNA encoding the mRNA, into the cell line; infecting the cell line with the first rotavirus; and recovering a reassortant rotavirus comprising a gene segment of a second rotavirus corresponding to said specific gene segment of a first rotavirus.

Another embodiment described herein provides an inhibitory RNA oligonucleotide that inhibits any one or more target sites selected from the group consisting of SEQ ID NO: 3, SEQ ID NO: 4, and SEQ ID NO: 5 for use in the method for producing a reassortant rotavirus as described above.

Another embodiment described herein provides a composition for use in the method for producing a reassortant rotavirus described above, the composition comprising an inhibitory RNA oligonucleotide that inhibits any one or more target sites selected from the group consisting of SEQ ID NO: 3, SEQ ID NO: 4, and SEQ ID NO: 5.

Another embodiment described herein provides a double-stranded DNA oligonucleotide formed by any one or more sequence pairs selected from the group consisting of SEQ ID NO: 6 and SEQ ID NO: 7, SEQ ID NO: 8 and SEQ ID NO: 9, and SEQ ID NO: 10 and SEQ ID NO: 11 for use in the method for producing a reassortant rotavirus as described above.

Another embodiment described herein provides a composition for use in the method for producing a reassortant rotavirus described above, the composition comprising a double-stranded DNA oligonucleotide formed by any one or more sequence pairs selected from the group consisting of SEQ ID NO: 6 and SEQ ID NO: 7, SEQ ID NO: 8 and SEQ ID NO: 9, and SEQ ID NO: 10 and SEQ ID NO: 11.

Another embodiment described herein provides a composition for producing a cell line with suppressed production of wild-type WC3 rotavirus, the composition comprising the inhibitory RNA oligonucleotide or the double-stranded DNA oligonucleotide.

Another embodiment described herein provides a composition for producing a cell line with suppressed production of bovine rotavirus VP7 protein, the composition comprising the inhibitory RNA oligonucleotide or the double-stranded DNA oligonucleotide.

Another embodiment described herein provides a cell line with suppressed production of wild-type WC3 rotavirus, wherein the cell line is introduced with the inhibitory RNA oligonucleotide or the double-stranded DNA oligonucleotide.

Another embodiment described herein provides a cell line with suppressed production of bovine rotavirus VP7 protein, wherein the cell line is introduced with the inhibitory RNA oligonucleotide or the double-stranded DNA oligonucleotide.

### [Advantageous Effects]

As the rotavirus reassortment method according to the present invention uses a reverse genetics method, the probability of reassortment is significantly higher than the conventional method of obtaining a reassortant virus by simultaneously infecting one cell line with two types of rotavirus. Therefore, it is expected that the time required can be greatly shortened. In addition, as monoclonal neutralizing antibodies are not used for selection of reassortant viruses, the cost and time required to obtain monoclonal neutralizing antibodies can be reduced. Therefore, using the present invention, the desired reassortant rotavirus can be produced in a shorter time and at a lower cost than conventional methods, and can be used as a vaccine.

### [Brief Description of Drawings]

Figures 1a to 1c show the alignment results of the nucleic acid sequences of the VP7 gene (SEQ ID NO: 1) of WC3 rotavirus and the VP7 gene (SEQ ID NO: 2) of Brb9 rotavirus. Gray shading indicates regions where the nucleic acids of the two genes are different, and underlined and bold text indicate three target sites selected according to one embodiment described herein.
Figure 2 shows a sequence of a miRNA target site selected from the VP7 gene of WC3 rotavirus, a top strand containing a DNA sequence encoding pre-miRNA for the miRNA capable of binding (hybridizing) to the target site and a bottom strand that is its complement, and a dsDNA oligo formed by combining the two strands with a 4nt overhang.
Figure 3 is a schematic diagram of a triple miRNA expression vector targeting the WC3 VP7 gene, according to one embodiment described herein.
Figure 4 is a schematic diagram of the design of a DNA template for the production of Brb9 VP7 mRNA, according to one embodiment described herein.
Figure 5 shows an RT-PCR result for confirmation of reassortment of P1 (Passage 1) virus according to one embodiment described herein.
Figure 6 shows an RT-PCR result for confirmation of reassortment of 10 clones isolated from P1 (Passage 1) by plaque isolation, according to one embodiment described herein. Lanes 1 to 10 represent each single clone; W represents WC3 viral RNA (PCR negative control); G represents G4 viral RNA (PCR positive control); M represents a 100bp DNA ladder.
Figure 7 shows a plaque of a G4 reassortant virus stock (reassortant virus stock obtained by culturing clones 6 and 8 identified in FIG. 6 in the MA-104 cell line) prepared by a reverse genetics method according to one embodiment described herein.
Figure 8 shows the reassortment confirmation results of the G4 reassortant virus stock using RT-PCR. Lane 1 and Lane 2 represent RT-PCR products obtained by culturing clones 6 and 8 identified in FIG. 6 in the MA-104 cell line, respectively, using the RNA of the reassortant virus as a template; Lane 3 represents a RT-PCR product performed using WC3 viral RNA (negative control) as a template; Land 4 represents a RT-PCR product (PCR reaction control) performed using a mixture of Brb9 synthetic DNA and WC3 viral RNA as a template.

### [Best Mode for Carrying Out the Invention]

According to one aspect of the present invention, there is provided a method for producing a reassortant rotavirus comprising: providing a cell line stably expressing an inhibitory RNA against a specific gene segment among 11 gene segments of a first rotavirus; introducing mRNA of a gene segment of a second rotavirus corresponding to said specific gene segment of the first rotavirus, or DNA or cDNA encoding the mRNA, into the cell line; infecting the cell line with the first rotavirus; and recovering a reassortant rotavirus comprising a gene segment of a second rotavirus corresponding to said specific gene segment of a first rotavirus.

In one embodiment, the first rotavirus may be a non-human rotavirus, for example bovine rotavirus, simian rotavirus, porcince rotavirus, canine rotavirus or goat rotavirus. As a preferred example, it may be a bovine WC3 rotavirus strain or a progeny thereof.

In one embodiment, the second rotavirus may be a human rotavirus, for example, a P1, P2, G1, G2, G3, G4 or G9 human rotavirus serotype. In addition, the second rotavirus may be WI79, Wa, D, WISC2, DS-1, WI78, P, HCR3A, Br (Bricout) B, ST-3, BrB-9, WI79-9, SC2-9, or WI78- 8 rotavirus strain, or a progeny thereof.

In one embodiment, the specific gene segment may be a gene segment encoding a protein selected from VP1, VP2, VP3, VP4, VP6, VP7, NSP1, NSP2, NSP3, NSP4, and NSP5, and as a preferred example, a gene segment encoding VP7 or VP4.

In one embodiment, the inhibitory RNA may be a miRNA, siRNA or shRNA.

In one embodiment, the inhibitory RNA may be two or more inhibitory RNAs targeting different target sites in the specific gene segment to be suppressed, and the two or more inhibitory RNAs may be contained in each vector or contained in one vector and introduced into a cell line.

In one embodiment, the inhibitory RNA may target at least one selected from the group consisting of target sites of SEQ ID NO: 3, SEQ ID NO: 4, and SEQ ID NO: 5.

In one embodiment, the inhibitory RNA may be miRNA, and the cell line may be introduced with a vector containing a double-stranded DNA oligonucleotide encoding a pre-miRNA for the miRNA.

In one embodiment, the double-stranded DNA oligonucleotide may be one or more double-stranded DNA oligonucleotides formed by one or more sequence pairs of selected from the group consisting of SEQ ID NO: 6 and SEQ ID NO: 7, SEQ ID NO: 8 and SEQ ID NO: 9, and SEQ ID NO: 10 and SEQ ID NO: 11.

In one embodiment, the vector may include a constitutive promoter.

In one embodiment, the vector may be a co-expression vector comprising two or more double-stranded DNA oligonucleotides encoding pre-miRNAs for two or more miRNAs having different target sites.

In one embodiment, mRNA of the gene segment of the second rotavirus corresponding to the specific gene segment of the first rotavirus may be obtained by in vitro transcription.

In one embodiment, the method for producing a reassortant rotavirus may further comprise enriching the reassortant rotavirus through subculture after the step of infecting the cell line with the first rotavirus.

In one embodiment, the cell line may be MRC-5, MA-104, Vero, BHK-21, COS-7, 293 T, CV-1 or TF-104 cell line.

In one embodiment, the method for producing a reassortant rotavirus may comprise providing a cell line stably expressing miRNA against a VP7 gene segment of bovine WC3 rotavirus; introducing mRNA of a VP7 gene segment of human rotavirus, or DNA or cDNA encoding the mRNA into the cell line; infecting the cell line with the WC3 rotavirus; and recovering a reassorted rotavirus comprising the VP7 gene segment of human rotavirus.

According to another aspect of the present invention, there is provided an inhibitory RNA oligonucleotide that inhibits any one or more target sites selected from the group consisting of SEQ ID NO: 3, SEQ ID NO: 4, and SEQ ID NO: 5.

In one embodiment, the inhibitory RNA may be a miRNA, siRNA or shRNA.

In another embodiment, the inhibitory RNA may be a miRNA or a pre-miRNA for the miRNA.

According to another aspect of the present invention, there is provided a double-stranded DNA oligonucleotide formed by any one or more sequence pairs selected from the group consisting of SEQ ID NO: 6 and SEQ ID NO: 7, SEQ ID NO: 8 and SEQ ID NO: 9, and SEQ ID NO: 10 and SEQ ID NO: 11

In another embodiment, the double-stranded DNA oligonucleotide is for use in the above-described method for producing a reassortant rotavirus.

According to another aspect of the present invention, there is provided a composition for producing a cell line with suppressed production of wild-type WC3 rotavirus, the composition comprising the above-described inhibitory RNA oligonucleotide or the double-stranded DNA oligonucleotide.

There is also provided a composition for producing a cell line with suppressed production of bovine rotavirus VP7 protein, the composition comprising the above-described inhibitory RNA oligonucleotide or the double-stranded DNA oligonucleotide.

According to another aspect of the present invention, there is provided a cell line with suppressed production of wild-type WC3 rotavirus, which is introduced with the above-described inhibitory RNA oligonucleotide or the double-stranded DNA oligonucleotide.

There is also provided a cell line with suppressed production of bovine rotavirus VP7 protein, which is introduced with the above-described inhibitory RNA oligonucleotide or the double-stranded DNA oligonucleotide.

Hereinafter, the present invention will be described in more detail.

Although any methods and materials similar or equivalent to those described herei n can be used in the practice or testing of the present invention, the preferred methods and materials are now described.

Unless defined otherwise, all technical and scientific terms used herein have the same meanings as commonly understood by one of ordinary skill in the art to which this invention belongs. It will be appreciated that the terms used herein and associated definitions are used for the purpose of explanation only and are not intended to be limiting.

As used herein, the singular forms are used herein to refer to one or more than one (i.e., at least one) of the grammatical objects. For example, "element" means one element or more than one element.

The term "about" used herein generally means within 20%, preferably within 10%, more preferably within 5% of a given value or range.

As described herein, "comprising (the specified components)" may mean that it may include additional components other than the listed components ("comprising"), or it may essentially include the listed components ("consisting essentially of').

One embodiment of the present invention provides a method for preparing a desired reassortant rotavirus by a reverse genetics method using a cell line constantly expressing an inhibitory RNA for a specific gene of rotavirus.

The reverse genetics method involves transfecting an excessive amount of mRNA of a gene to be reassorted or DNA that can be transcribed into the mRNA into a cell and simultaneously infecting the cell with helper rotavirus in order to induce reassortment of the transfected mRNA and the genes of the helper rotavirus. In one embodiment of the present application, which will be described later, provides an example in which the bovine WC3 rotavirus strain was used as a helper virus, and VP7 coding RNA segment, which is RNA segment 9 of the human G4 rotavirus strain BrB9 (Bricout B 9), was used as the gene to be transfected for reassortment, and thus WC3/Brb9-VP7 reassortant virus was produced as a result of reassortment of the helper virus and the transfected gene. However, such an example is only for illustrating the present invention, and the scope of the present invention is not limited by the example.

In the present application, a cell line constantly expressing an inhibitory RNA for a specific gene of rotavirus provides a selective pressure for selecting and isolating a desired reassortant rotavirus. In particular, the conventional method of applying selective pressure using a monoclonal neutralizing antibody requires a lot of time and cost to obtain the neutralizing antibody and has the problem of making it difficult to obtain a neutralizing antibody specific to the protein of a specific strain because rotavirus strains are composed of genes with similar sequences, functions, and structures. However, the present invention has advantages as follows: as the target sequence of the inhibitory RNA is short (about 21bp), it is easy to find different parts even between genes of similar strains, making it easy to apply selection pressure. Further, when multiple target sites are used, stronger selection pressure can be applied. Moreover, cell lines that constantly express an inhibitory RNA for a specific gene of rotavirus can be easily prepared.

In a preferred embodiment, the method for producing a reassortant rotavirus includes the following steps:
providing a cell line stably expressing an inhibitory RNA against a specific gene segment among 11 gene segments of a first rotavirus;
introducing mRNA of a gene segment of a second rotavirus corresponding to said specific gene segment of the first rotavirus, or DNA or cDNA encoding the mRNA, into the cell line;
infecting the cell line with the first rotavirus; and
recovering a reassortant rotavirus comprising a gene segment of a second rotavirus corresponding to said specific gene segment of a first rotavirus.

In one embodiment, if the specific gene is a single gene, the produced reassortant rotavirus will have 11 gene segments, with the gene segment corresponding to the specific gene segment being from the second rotavirus and the remaining 10 gene segments being from the first rotavirus.

In another embodiment, if the specific gene is "n" genes, the produced reassortant rotavirus will have 11 gene segments, with the "n" gene segments corresponding to the specific gene segments being from the second rotavirus and the remaining "11-n" gene segments being from the first rotavirus.

Rotaviruses generally have a genome composed of 11 gene segments: 6 gene segments each encoding structural proteins (VP1, VP2, VP3, VP4, VP6, and VP7) and 5 gene segments each encoding non-structural proteins (NSP1, NSP2, NSP3, NSP4, and NSP5). Among them, VP7 and VP4 are structural proteins present on the surface and determine the serotype of the virus. In particular, VP7 determines the G serotype and VP4 determines the P serotype. Therefore, the serotype of rotavirus is indicated as G type and P type together. For type G, as the serotype and genotype are the same, the serotypes are denoted by a number (e.g., G1, G2, G3), and for type P, as the serotype and genotype are not the same, the serotypes are denoted by an open number and the genotypes are indicated by closed bracket (e.g., P1A[8], P1B[4]), or the genotype is indicated alone (e.g., P[8], P[4]).

In one embodiment, the first rotavirus may be a non-human rotavirus and the second rotavirus may be a human rotavirus. This is based on the idea that non-human rotaviruses may exhibit reduced virulence in humans. However, the present invention is not limited thereto, and both the first rotavirus and the second rotavirus may be non-human rotaviruses, or both may be human rotaviruses.

In a preferred embodiment, the first rotavirus may be a non-human rotavirus, such as bovine rotavirus, simian rotavirus, porcince rotavirus, canine rotavirus or goat rotavirus. Examples of bovine rotavirus include rotavirus strain NCDV, WC3 or UK, etc. Examples of simian rotavirus include rhesus monkey rotavirus (RRV) strain TUCH, MMU 18006 or MMU17959, vervet monkey monkey rotavirus strain SA11, or pig-tailed macaque rotavirus strain PTRV (Macaca nemestrina) or YK-1 (macaques), etc., but are not limited thereto.

In another preferred embodiment, the second rotavirus may be a human rotavirus, which may have any P serotype or G serotype, but is preferably P1, P2, G1, G2, G3, G4 or G9 serotype, which is the serotype primarily causing the disease. Examples of human rotavirus of G1 serotype include rotavirus strains WI79, Wa, D, etc., examples of human rotavirus of G2 serotype include rotavirus strains WISC2, DS-1, etc., examples of human rotavirus of G3 serotype include rotavirus strains WI78, P, HCR3A, etc., examples of human rotavirus of G4 serotype include rotavirus strains Br(Bricout) B, ST-3, etc., examples of human rotavirus of G9 serotype include rotavirus strain WI61, etc., examples of human rotavirus of P1 serotype include rotavirus strains WI79, WI78, WI61, Wa, etc., and examples of human rotavirus of P2 serotype include rotavirus strain DS-1, etc., but are not limited thereto.

Alternatively, the first rotavirus or the second rotavirus or both may be previously known reassortant viruses, for example, BrB-9 (GenBank Accession No. GU565093), WI79-9 (GenBank Accession No. GU565060), SC2-9 (GenBank Accession No. GU565071), WI78-8 (GenBank Accession No. GU565082), or WI79-4 (GenBank Accession No. GU565049), but is not limited thereto.

As used herein, inhibitory RNA refers to a nucleic acid molecule that can sequence-specifically inhibit or reduce the expression of a target gene. The inhibitory RNA may be sufficiently complementary to the target gene or its mRNA to bind (hybridize) to the target gene or its mRNA to inhibit or reduce transcription and/or expression. The inhibitory RNA may inhibit or reduce the transcription and/or expression of a target gene through RNA interference (RNAi). In a preferred embodiment, the inhibitory RNA may be short interfering RNA (siRNA), short hairpin RNA (shRNA) or microRNA (miRNA).

RNAi refers to a phenomenon in which gene expression is sequence-specifically inhibited by short-length (usually 12 to 21 mer) double-stranded RNA (dsRNA). These dsRNAs can selectively degrade target mRNAs complementary thereto, thereby stopping transcription and/or protein synthesis of the target gene. RNAi is therefore widely used to silence genes of interest. siRNA and miRNA, which are representative small RNAs that cause RNAi, are produced when dicer enzyme, a dsRNA-specific endonuclease belonging to the RNase III group, cleaves dsRNA precursors.

siRNA is a dsRNA composed of about 21 nucleotides, consisting of about 19 base pairs, and two nucleotide overhangs on the 3' side. These 3' overhangs serve as intermediate mediators in the RNAi process. siRNA is produced when long dsRNA is produced from transposons, viruses, or in vivo genes, or when dsRNA is artificially transfected into cells from the outside. Then, the siRNA is separated into a single strand and included in RNA induced silencing complex (RISC), and after being integrated into RISC, it forms a complementary pair to the target mRNA, and cleaves and degrades mRNA or inhibits mRNA translation depending on the degree of complementarity with the target mRNA. RNAi using siRNA can exhibit the effect of suppressing gene expression by a specific nucleic acid sequence by directly transfecting cells with dsRNA against the target sequence in vitro.

In the case of RNAi using shRNA, instead of directly transfecting cells with synthetic dsRNA, plasmid DNA that transcribes single-stranded RNA having a hairpin structure is used. Specifically, the plasmid controlled by the RNA polymerase III promoter is transcribed into a short hairpin-shaped single-stranded RNA in the nucleus of the target cell, then moved to the cytoplasm by exportin-5 and processed by dicer, and thus the shRNA is converted into siRNA.

miRNAs are naturally produced in cells, and are RNAs that do not have genetic information to produce proteins.

miRNA is an RNA that is naturally occurring in cells and does not contain the genetic information to produce proteins. The formation process of miRNA is as follows: In the nucleus, primary miRNA (pri-miRNA) is transcribed from the genome and then excised by drosha to form a short hairpin-shaped precursor miRNA (pre-miRNA), which exported from the nucleus to the cytoplasm by exportin-5. In the cytoplasm, pre-miRNA is cleaved by dicer enzyme into dsRNA of approximately -22 nt with a 2 nt overhang structure on the 3' side, and then included in RISC to form a single-stranded mature miRNA. The mature miRNA can form a complementary pair to the target mRNA, and depending on the degree of complementarity with the target mRNA, it can cleave and degrade the mRNA or inhibit mRNA translation.

In one embodiment, the inhibitory RNA herein may be an engineered miRNA, which is fully complementary to the target site and can cleave the target mRNA. The engineered miRNA may be introduced into cells through a vector containing DNA encoding the same and stably expressed within the cell.

The vector is transcribed after being introduced into a cell to express pre-miRNA. The pre-miRNA forms a stem-loop structure in the form of a hairpin similar to the endogenous pre-miRNA structure, and the expression and action mechanism of miRNA as described above It is converted into a mature form of miRNA through cleavage and degradation of the target mRNA, thereby suppressing the expression of the target gene. "Stable expression" refers to constant, constitutive, or permanent expression of a nucleic acid of interest, as opposed to transient expression.

In a preferred embodiment, the inhibitory RNA herein is an engineered miRNA and can be introduced into a cell via a vector containing a double-stranded DNA oligonucleotide encoding a pre-miRNA engineered for the miRNA. After being introduced into cells, the vector is transcribed to express pre-miRNA, which forms a hairpin-shaped stem-loop structure similar to the endogenous pre-miRNA structure. The pre-miRNA is converted into a mature form of miRNA through above-described miRNA expression and action mechanism, cleaving and degrading the target mRNA, thereby suppressing the expression of the target gene.

In one embodiment, the double-stranded DNA oligonucleotide encoding the pre-miRNA may be constructed as follows: (i) a 5' terminal overhang for cloning into vector; (ii) an antisense sequence (i.e., mature miRNA sequence) coding region complementary to the target region; (iii) a spacer sequence to form a terminal loop structure; (iv) a sequence coding region (i.e., sense sequence for target site) complementary to the region (ii) to form a stem structure; (v) a 3' terminal overhang for cloning into vector. Optionally, in the region (iv), some nucleotides (e.g., about 2 nt) may be deleted to form a short internal loop in order to increase the efficiency of suppressing gene expression.

In addition, the double-stranded DNA oligonucleotide may be composed of a top strand constructed as above and a bottom strand that is the complement of the top strand.

In one embodiment, the vector herein may include a constitutive promoter to stably express the inhibitory RNA. The promoter may be an RNA polymerase II-dependent promoter. In addition, the vector may include conventional expression control sequences. For example, adapters or linkers, enhancers, selectable markers (e.g., antibiotic resistance markers), replicable units, polyadenylation (polyA) signal sequences, tags for purification, or any known sequences for regulating the expression of genes or inducing the expression in prokaryotic or eukaryotic cells or viruses, and various combinations thereof may be appropriately included.

The efficiency of the RNAi effect of miRNA is determined by the target sequence. Over the past few years, an algorithm has been developed to design functional miRNA based on the database that has investigated the base sequences of numerous miRNAs and the resulting RNAi efficiency, and is available on several websites (e.g., www.ambion.com, www.oligoengine.com, www.vectorcorea.com).

However, the miRNA determined through the algorithm is not necessarily capable of effectively degrading the target mRNA, and one target site alone may be insufficient for efficient inhibition. Thus, manufacturing miRNA by selecting two or more target sites per gene can increase the probability of success in gene suppression experiments.

Accordingly, the vectors herein may be two or more vectors each containing two or more double-stranded DNA oligonucleotides encoding pre-miRNAs for two or more miRNAs having different target sites; or may be a co-expression vector containing two or more double-stranded DNA oligonucleotides encoding pre-miRNAs for two or more miRNAs having different target sites.

The vector may be a plasmid vector known in the art such as pcDNA (Invitorgen) or a viral vector, but is not limited thereto. The introduction into a cell is performed by a method known in the art, for example, the calcium phosphate method, or the method using various transfection reagents (e.g., oligofectamine, lipofectamine, or lipofection, etc.) can be appropriately performed.

As a non-limiting example according to one embodiment of the present invention, the inhibitory RNA may target one or more target sites selected from the group consisting of SEQ ID NO: 3, SEQ ID NO: 4, and SEQ ID NO: 5.

The above three target sites were selected by screening nucleotide sequence regions with low homology (%) between the nucleotide sequences of the first rotavirus and the second rotavirus. If an inhibitory RNA targeting a different target site is used, the sequence homology (%) increases, and thus the effect of suppressing expression on the target site may be lower or absent.

As a non-limiting example according to one embodiment of the present invention, the double-stranded DNA oligonucleotide may be one or more double-stranded DNA oligonucleotides formed by one or more sequence pairs of selected from the group consisting of SEQ ID NO: 6 and SEQ ID NO: 7, SEQ ID NO: 8 and SEQ ID NO: 9, and SEQ ID NO: 10 and SEQ ID NO: 11.

A cell line used herein that stably expresses an inhibitory RNA against a specific gene segment among 11 gene segments of a first rotavirus may be a cell with high sensitivity to rotavirus. For example, the cell line may be MRC-5, MA-104, Vero, BHK-21, COS-7, 293 T, CV-1 or TF-104 cell line, but is not limited thereto.

Meanwhile, in the method for producing a reassortant virus herein, the mRNA, DNA or cDNA of a gene segment of a second rotavirus corresponding to the specific gene segment of the first rotavirus is introduced into the cell line.

When mRNA of a gene segment of a second rotavirus corresponding to the specific gene segment of the first rotavirus is introduced into a cell, the mRNA may be synthesized by methods known in the art or obtained by in vitro transcription. In this case, considering that natural rotavirus mRNA has a cap structure at the 5' end, which affects genome replication and translation, it is preferable to cap the obtained mRNA.

Alternatively, when DNA or cDNA encoding an RNA segment of a second rotavirus corresponding to the specific gene segment of a first rotavirus is introduced into a cell, the T7 promoter, which is recognized by T7 RNA polymerase, may be generally used. However, other promoters recognized by various RNA polymerases may also be used, for example, T3 RNA polymerase or Sp6 RNA polymerase, but are not limited thereto. The DNA or cDNA can be introduced using a known vector. For example, as a T7 RNA polymerase promoter, a known T7 expression vector such as commercially available pBluescriptII SK (+/-) (Stratagene), pCRII (Invitrogen), pGEM (Promega), pET (Novagene), pcDNA (Invitorgen), etc. may be used, but is not limited thereto. In addition, when DNA or cDNA is introduced into cells as described above, a nucleic acid construct expressing RNA polymerase may be introduced before or after introducing DNA or cDNA into the cell. Preferably, the nucleic acid construct used to supply RNA polymerase may include a sequence encoding a capping enzyme in an expressible state.

After the genetic segment of the second rotavirus is introduced into the cell, the cell is infected with the first rotavirus as a helper virus. The helper virus may be a wild-type virus that exists in nature, but is not limited thereto, and may also be a mutant virus that exists in nature or a recombinant virus that has undergone artificial genetic modification. In a preferred embodiment, infection may be performed in the presence of trypsin or chymotrypsin to facilitate infection.

After infecting the cell line with the first rotavirus, the method for producing a reassortant rotavirus herein may further include enriching the reassortant rotavirus through subculture.

As the method herein uses a cell line that constantly expresses inhibitory RNA against a specific rotavirus gene, selective pressure is applied to select and isolate reassortant rotaviruses. Specifically, in the cells used herein, the mRNA level of the specific gene segment of the first rotavirus is reduced by inhibitory RNA, while an excessive amount of the corresponding specific gene segment of the second rotavirus is introduced. Therefore, during the replication and assembly process of the virus, the mRNA of the specific gene segment of the second rotavirus is used instead of the mRNA of the specific gene segment of the first rotavirus, thereby increasing the probability of occurrence of a reassortant virus. Further, as passage continues, the replication of helper virus (e.g., wild-type WC3 virus) continues to be inhibited by the miRNA expressed by cells, but reassortant virus is not affected, and thus, reassortant viruses may be enriched.

The recovery of the reassortant virus may be performed by recovering the culture medium and/or the cell lysate after culturing. Optionally, the virus may be re-infected by adding (inoculating) the recovered culture medium and/or cell lysate to another host cell (second host cell, third host cell), subcultured, and then recovered. Virus recovery may be performed through plaque isolation, and the recovered recombinant virus may be purified if necessary. For example, viruses may be purified by appropriately combining centrifugation, density gradient centrifugation, column chromatography, and the like.

An example according to one embodiment of the present invention provides a method for producing a reassortant rotavirus comprising: providing a cell line stably expressing miRNA against a VP7 gene segment of bovine WC3 rotavirus; introducing mRNA of a VP7 gene segment of human rotavirus, or DNA or cDNA encoding the mRNA into the cell line; infecting the cell line with the WC3 rotavirus; and recovering a reassorted rotavirus comprising the VP7 gene segment of human rotavirus. The detailed descriptions of each step are as described above.

The present invention also relates to an inhibitory RNA oligonucleotide that inhibits any one or more target sites selected from the group consisting of SEQ ID NO: 3, SEQ ID NO: 4, and SEQ ID NO: 5. In a preferred embodiment, the inhibitory RNA may be miRNA, siRNA or shRNA. In another preferred embodiment, the inhibitory RNA may be a miRNA or a pre-miRNA for the miRNA.

The present invention also provides a double-stranded DNA oligonucleotide formed by any one or more sequence pairs selected from the group consisting of SEQ ID NO: 6 and SEQ ID NO: 7, SEQ ID NO: 8 and SEQ ID NO: 9, and SEQ ID NO: 10 and SEQ ID NO: 11; a composition for producing a cell line with suppressed production of wild-type bovine WC3 rotavirus, the composition comprising the inhibitory RNA oligonucleotide or the double-stranded DNA oligonucleotide; a composition for producing a cell line with suppressed production of bovine rotavirus VP7 protein, the composition comprising the inhibitory RNA oligonucleotide or the double-stranded DNA oligonucleotide; a cell line with suppressed production of wild-type WC3 rotavirus, which is introduced with the inhibitory RNA oligonucleotide or the double-stranded DNA oligonucleotide; and a cell line with suppressed production of bovine rotavirus VP7 protein, which is introduced with the above-described inhibitory RNA oligonucleotide or the double-stranded DNA oligonucleotide. They can be used in the method for producing a reassortant virus as described above.

### [Detailed Description of the Embodiments]

Hereinafter, the present invention will be described in more detail with reference to the following examples. However, these examples are only for illustrating the present invention, and the scope of the present invention is not limited by these examples. It is apparent to those skilled in the art that the examples described below may be modified without departing from the essential gist of the disclosure.

### Example 1. Selection of target sites for inhibition of transcription and expression of VP7 gene in WC3 wild-type rotavirus

To produce WC3/Brb9-VP7 reassortant virus, cells with high susceptibility to rotavirus were transformed to establish a stable cell line capable of suppressing the production of unrearranged bovine WC3 wild type virus. The transformation used RNA interference (RNAi) technology targeting the VP7 gene of the WC3 virus.

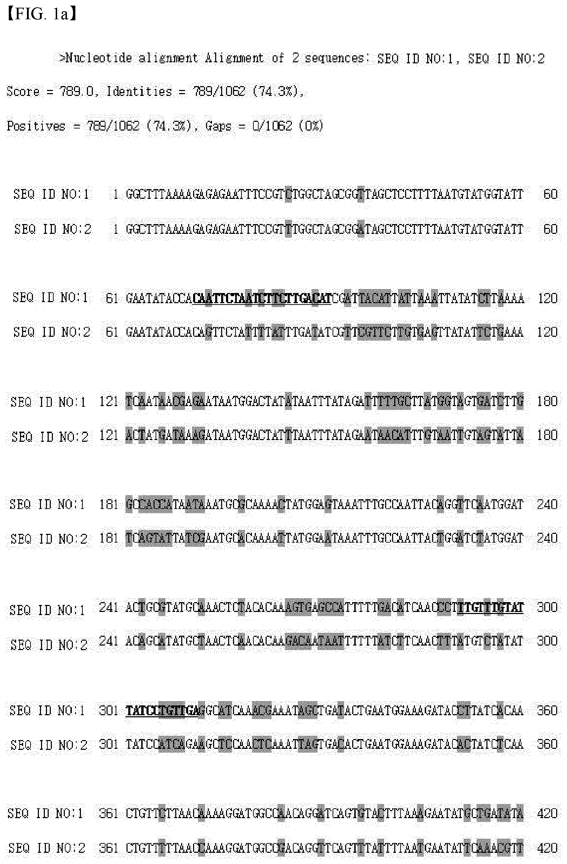

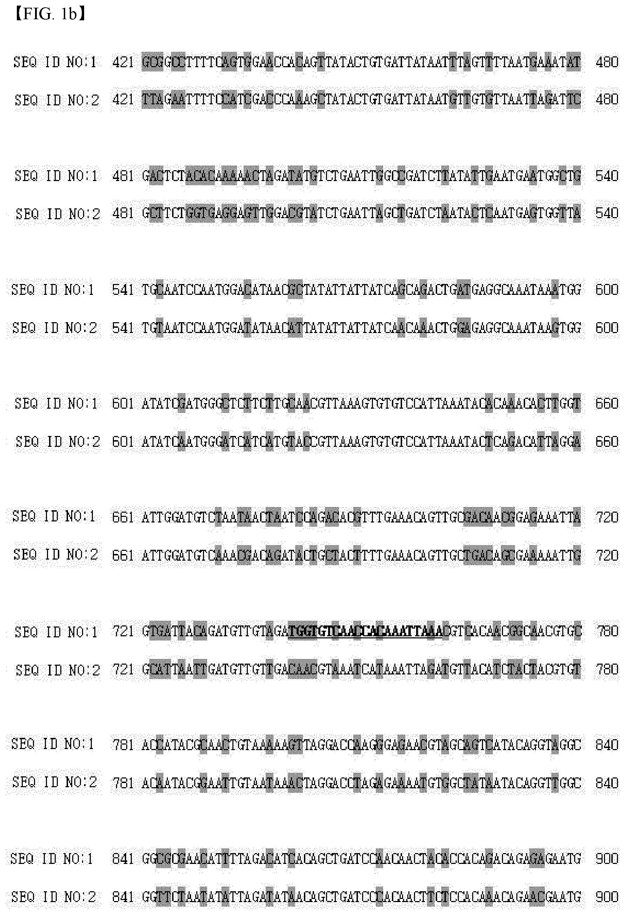

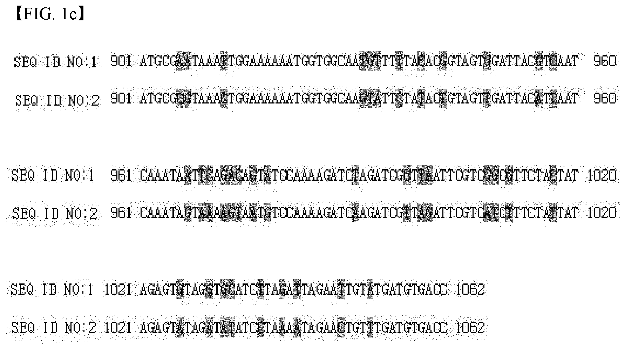

Specifically, within the VP7 gene of WC3 rotavirus, three miRNA target sites that are specific to WC3 but not specific to VP7 of a human rotavirus BrB9 were selected. The stable cell lines to be established herein are intended to constitutively express miRNAs for these target sites. The miRNA (mature form) is a type of short interfering RNA of 21 bp that corresponds to an antisense sequence that is completely complementary to the RNAi target site, and inhibits the transcription and expression of the WC3 VP7 gene. To select miRNAs, Invitrogen's Stealth RNAi^{™} Pre-Designed siRNAs web-tool (www.invitrogen.com/rnaiexpress) provided by the vector system used for RNAi expression was used. GenBank AY050272 (SEQ ID NO: 1) was referenced for the WC3 VP7 gene, and GenBank GU565090 (SEQ ID NO: 2) was referenced for the Brb9 VP7 gene. Figure 1 show the alignment results of the nucleic acid sequences of the WC3 VP7 gene (SEQ ID NO: 1) and the Blb9 VP7 gene (SEQ ID NO: 2). As shown in FIG. 1, the homology between the nucleic acid sequences of the WC3 VP7 gene (SEQ ID NO: 1) and the Blb9 VP7 gene (SEQ ID NO: 2) is 74.3% (789 bp of 1062 bp identical), and the target site was selected from the region showing lower homology. Specifically, in Figure 1, the three selected target sites are underlined and indicated in bold. In order from 5' to 3' direction, target site 1 (SEQ ID NO: 3) has a sequence homology of 71.4% (15/21), target site 2 (SEQ ID NO: 4) has a sequence homology of 66.7% (14/21), and target site 3 (SEQ ID NO: 5) has a sequence homology of 61.9% (13/21).

### Example 2. Construction of miRNA expression vectors

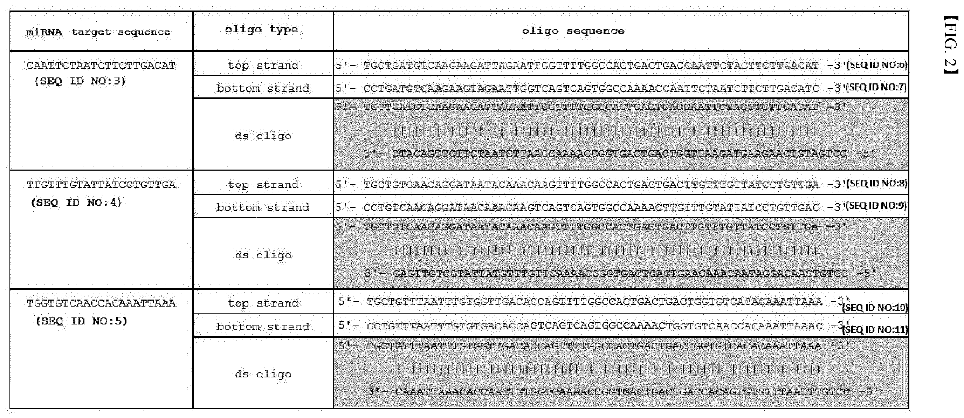

In order to express the miRNAs against the three miRNA target sites selected in Example 1, the sequences encoding for their pre-miRNA were cloned into each miRNA expression vector, pcDNA6.2-GW/EmGFP-miR (Invitrogen), according to the manufacturer's manual. To this end, first, a DNA sequence that can transcribe pre-miRNA for each miRNA capable of binding to each target site as well as its complementary sequence were designed and synthesized to have an overhang of 4 nt and then annealed to produce a dsDNA oligo (Table 1 and Fig. 2).

**[Table 1]**

| miRNA target site | oligo type | oligo sequence (5'->3') | SEQ ID NO: |
|---|---|---|---|
| target site 1 (SEQ ID NO:3) | top strand | TGCTGATGTCAAGAAGATTAGAATTGGT TTTGGCCACTGACTGACCAATTCTACTT CTTGACAT | 6 |
| | bottom strand | CCTGATGTCAAGAAGTAGAATTGGTCA GTCAGTGGCCAAAACCAATTCTAATCTT CTTGACATC | 7 |
| target site 2 | top strand | TGCTGTCAACAGGATAATACAAACAAG | 8 |
| (SEQ ID NO.4) | | TTTTGGCCACTGACTGACTTGTTTGTTAT CCTGTTGA | |
| | bottom strand | CCTGTCAACAGGATAACAAACAAGTCA GTCAGTGGCCAAAACTTGTTTGTATTAT CCTGTTGAC | 9 |
| target site 3 (SEQ ID NO: 5) | top strand | TGCTGTTTAATTTGTGGTTGACACCAGT TTTGGCCACTGACTGACTGGTGTCACAC AAATTAAA | 10 |
| | bottom strand | CCTGTTTAATTTGTGTGACACCAGTCAG TCAGTGGCCAAAACTGGTGTCAACCAC AAATTAAAC | 11 |

Then, according to the manufacturer's manual, the dsDNA oligo with a 4nt overhang and the linearized pcDNA6.2-GW/EmGFP-miR vector (Invitrogen) were ligated, transformed into E.coli, and then colonies were selected and sequence analysis was performed to obtain complete miRNA expression plasmids, which were named pcDNA-GFP/mr1, pcDNA-GFP/mr2, and pcDNA-GFP/mr3, respectively.

### Example 3. Construction of triple miRNA expression vectors

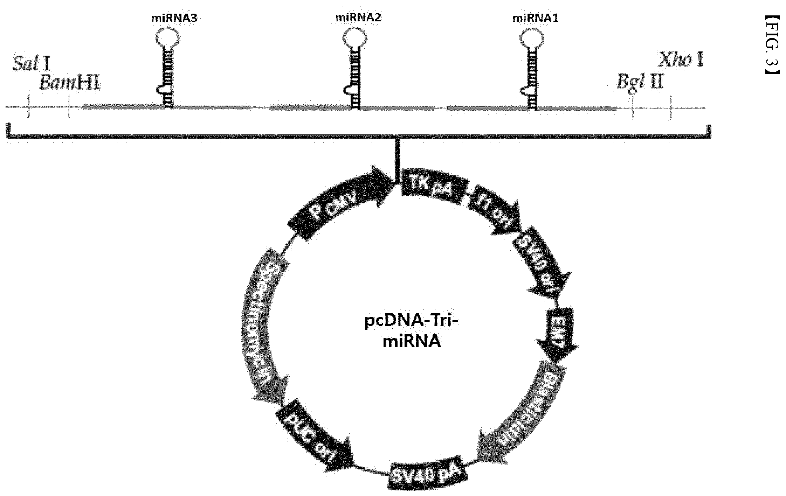

An expression vector capable of simultaneously transcribing three miRNAs in a single vector was designed and cloned according to the manufacturer's manual. First, pcDNA-GFP/mr1 was digested with BamHI and XhoI restriction enzymes, and an insert fragment of approximately 151 bp was purified. pcDNA-GFP/mr2 was digested with BglII and XhoI restriction enzymes, and a vector fragment of approximately 5.6 kb was purified. The insert fragment and the vector fragment were ligated and transformed into E. coli, and then colonies were selected and sequence analysis was performed to prepare a complete dual miRNA expression vector. Then, the double miRNA expression vector was digested with BamHI and XhoI restriction enzymes, and insert fragment 2 of approximately 250 bp was purified, and pcDNA-GFP/mr3 was digested with BglII and XhoI restriction enzymes, and vector fragment 2 of approximately 5.6 kb was purified. The insert fragment 2 and the vector fragment 2 were ligated and transformed into E. coli, and then colonies were selected and sequence analysis was performed to prepare a complete triple miRNA expression vector. The prepared triple miRNA expression vector was digested with DraI restriction enzyme, the EmGFP fragment was removed, and then ligated again to construct a triple miRNA expression vector without GFP gene, which was named pcDNA-Tri-miRNA (Fig. 3).

### Example 4. Construction of cell lines stably expressing triple miRNAs and WC3 infection inhibition test

In order to obtain a cell line in which triple miRNA is stably expressed, TF-104 (African green monkey epithelial cell line) cell line was transfected with the pcDNA-Tri-miRNA plasmid, and single cells expressing miRNA were selected using an antibiotic resistance gene for blasticidin, which was contained in the vector. Transfected cells were treated with the antibiotic, Blasticidin S at a concentration of 10-20 ug/ml, and the process of inhibiting the growth of non-transfected cells and selecting transfected cells was repeated several times. As a result, among the single cell colonies resistant to blasticidin, 44 colonies were expanded and passaged to obtain 44 clones.

Each of the above clones and wild-type (WT) TF-104 cells were plated at 90% confluency in a 24-well plate and infected with WC3 virus at 100 pfu and 1 pfu per well. Cytopathic effect (CPE) was compared 3 days and 5 days after infection, and clones with inhibited infection compared to WT TF-104 were selected. As a result, as shown in Table 2, clones 6, 13, 14, 19, 31, and 44 effectively inhibited WC3 infection, and in particular, clone 44 showed the greatest inhibitory effect.

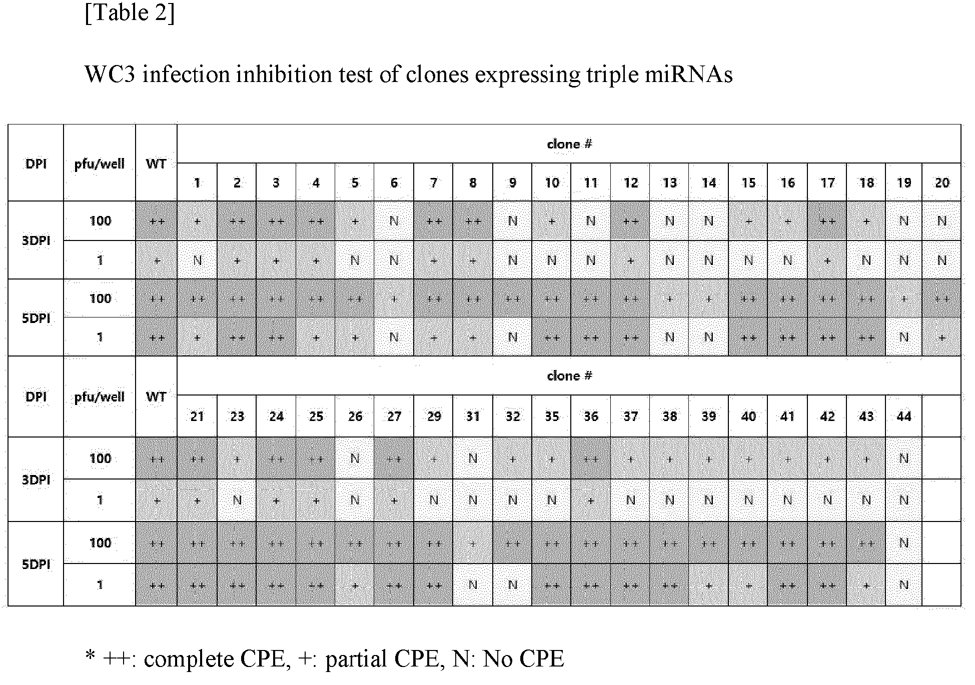

The media of clones 6, 13, 14, 19, 31, and 44 infected with WC3 virus were removed, replaced with EMEM media containing 10% FBS and 10ug/ml blasticidin, and extended subculture was performed to rescue cells that were not infected with the virus. The cells were named as TK6r, TK13r, TK14r, TK19r, TK31r, and TK44r, respectively.

### Example 5. Construction of G4 Brb9 VP7 mRNA

The VP7 gene of human rotavirus G4 Brb9 to be used for reassortment was transcribed into mRNA through in vitro transcription and capping processes, and then the mRNA was transduced. It can be transduced directly in the form of plasmid DNA, but in this case, the T7 promoter is generally used for efficient transcription. As the T7 promoter is specific and strong and can be transcribed even in the cytosol, it has the advantage of allowing transcription even if the plasmid remains in the cytoplasm rather than being delivered into the nucleus. However, this has the disadvantage that an expression vector capable of expressing T7 polymerase must first be transduced into cells and that RNA transcribed in the cytoplasm is unstable because it is not capped. In order to increase the reassortment efficiency of the Blb9 VP7 gene, it is advantageous to use a capped form that is the same as the viral mRNA. Thus, in this example, instead of transducing plasmid DNA, transcription was performed in vitro to have the same form as the viral mRNA, and this mRNA was introduced.

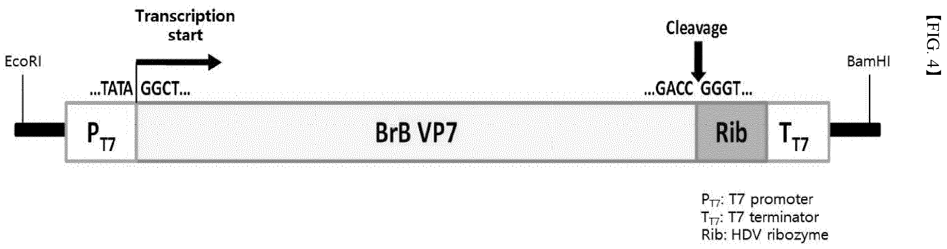

A DNA template was designed to transcribe the VP7 gene of G4 Brb9 into the mRNA in vitro. As 3'UTR sequence of rotavirus is important for the translation process, transcription must start and end at the correct location. Although the starting point of transcription of the T7 promoter is very precise, the ending point is somewhat heterogeneous even if the T7 terminator is present. Therefore, the DNA template was designed to be cut accurately at the position where the VP7 gene ends by introducing the delta hepatitis virus (HDV) ribozyme sequence in front of the T7 terminator (See SEQ ID NO: 12 and Fig. 4). The designed DNA template was synthesized, digested with EcoRI/BamHI restriction enzymes, cloned into pUC57 vector, and named pUC57-T7-Brb9-VP7.

Using the mMESSAGE mMACHINE kit (Ambion) and the pUC57-T7-Brb9-VP7 DNA template, Brb9 VP7 mRNA was produced through in vitro transcription and 5' capping according to the manufacturer's manual.

### Example 6. G4 Brb9 VP7 mRNA Transfection and WC3 Virus Infection

The in vitro transcribed G4 Blb9 VP7 mRNA was liposome (Lipofectamine 2000; Thermo Fisher)-transfected into the TK14r cell line obtained in Example 4. The transfection process was performed according to a conventional method provided by the manufacturer's manual. After transfection, the cells were cultured in an incubator at 37°C, 5% CO₂ for 4 hours, and then the medium was replaced with complete growth medium (EMEM containing 10% FBS).

After 24 hours of transfection, the medium was replaced with infection medium (serum-free EMEM containing 0.5 µg/ml trypsin), and WC3 virus (ATCC VR-2102) activated using trypsin (Sigma, T0303, 10µg/mℓ) at 37°C for 30 minutes was infected with cells at an MOI of 1.0. After infection, the cells were incubated for 48 hours in an incubator at 37°C, 5% CO₂, and the cytopathic effect (CPE) was confirmed.

In the TK14r cells, the level of WC3 VP7 mRNA is reduced by suppressing the transcription and expression of the WC3 VP7 gene using miRNA, while an excessive amount of BrB9 VP7 mRNA was introduced. Therefore, during the replication and assembly process of the virus, the BrB9 VP7 mRNA is used instead of the WC3 VP7 mRNA, thereby increasing the probability of occurrence of a reassortant virus. Further, as passage continues, the replication of the WC3 WT virus continues to be inhibited by the miRNA expressed by cells, but reassortant virus is not affected, and thus, reassortant viruses can be enriched.

### Example 7. Identification of WC3/Brb9 VP7 reassortant virus

Through the above described process, the cells in which CPE had occurred were freeze-thawed 3 times, cell debris was removed, and the supernatant was obtained, which was designated as Passage 0 (P0). 48 hours after P0 was reinfected with the TK14r cell line or MA-104 cell line, the cells were freeze-thawed three times, cell debris was removed, and the supernatant was obtained, which was designated as Passage 1 (P1).

In order to confirm the reassortment in the recombinant sample, primer sets that can specifically detect WC3 VP7, BrB9 VP7, and WC3 VP4 were prepared. No cross activity or false positive and false negative results were observed (Table 3).

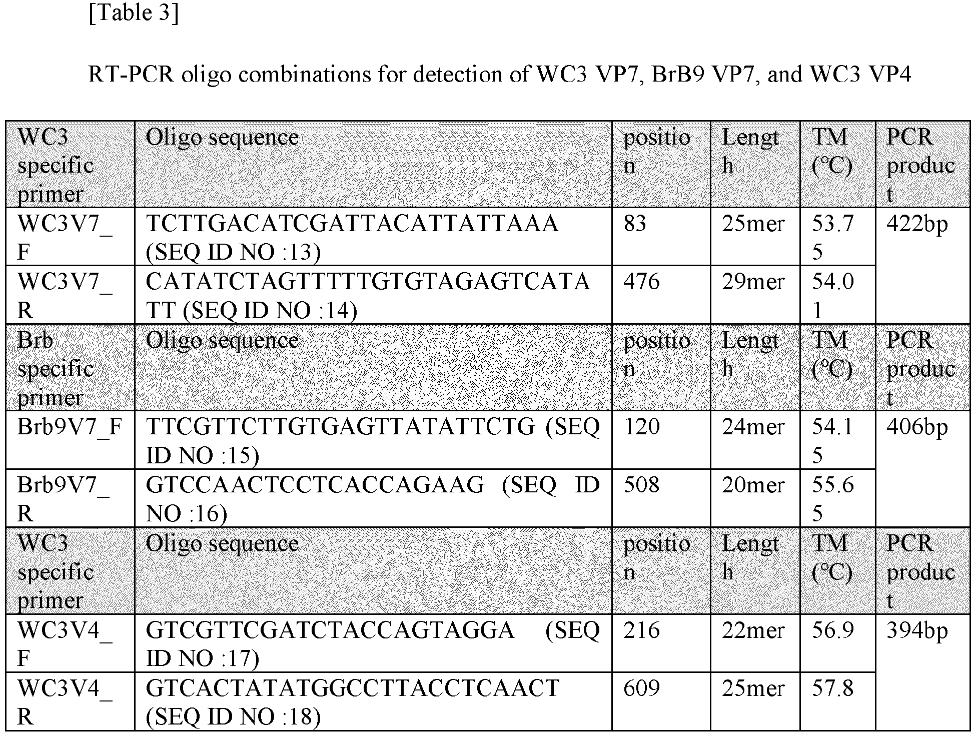

An RT-PCR reaction was performed to confirm the reassortment in the sample using the above primers. WC3 VP7, BrB9 VP7, and WC3 VP4 were amplified as RT-PCR products of 422 bp, 406 bp, and 394 bp, respectively, and the RT-PCR conditions used were as follows (Table 4).

**[Table 4]**

| RT-PCR reaction conditions for detection of VP7 and VP4 genes | | | | | |
|---|---|---|---|---|---|
| VP7 RT-PCR condition | | | VP4 RT-PCR condition | | |
| 1) cDNA synthesis and pre-denaturation | | | 1) cDNA synthesis and pre-denaturation | | |

| Cycle | Temp | Time | Cycle | Temp | Time |
|---|---|---|---|---|---|
| 1 | 50°C | 45min | 1 | 54°C | 45min |
| | 95°C | 2min | | 95°C | 2min |
| 2) PCR amplification | | | 2) PCR amplification | | |

| Cycle | Temp | Time | Cycle | Temp | Time |
|---|---|---|---|---|---|
| 40 | 94°C | 30sec | 40 | 94°C | 30sec |
| | 54°C | 40sec | | 57.5°C | 40sec |
| | 68°C | 40sec | | 68°C | 40sec |
| 1 | 68°C | 7min | 1 | 68°C | 7min |
| 1 | 4°C | ∞ | 1 | 4°C | ∞ |

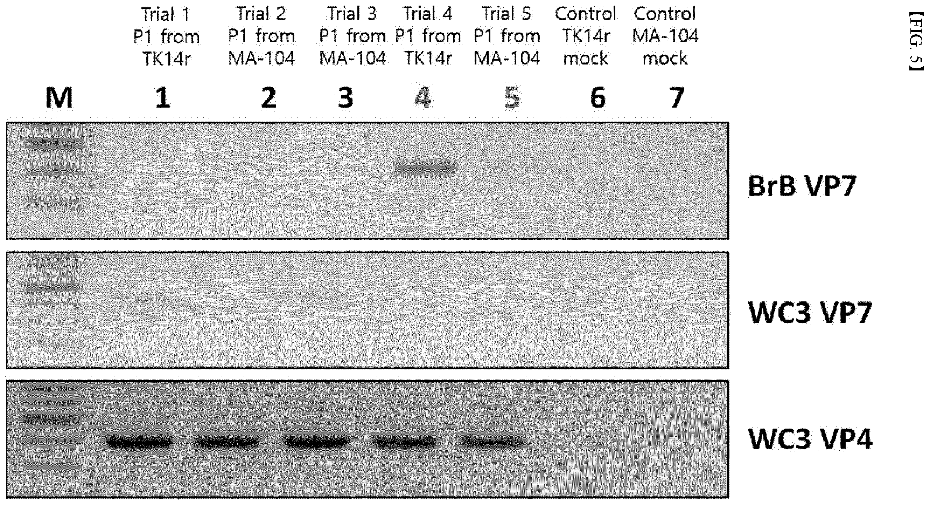

RT-PCR was performed by extracting viral RNA from two P1 samples obtained after infecting the TK14r cell line or the MA-104 cell line with the P0 sample. Of the five attempts, P1 was prepared using the TK14r cell line twice (Fig. 5, Lanes 1 and 4), and P1 was prepared using the MA-104 cell line three times (Fig. 5, Lanes 2, 3, and 5). As a result, a band of approximately 400bp in size specific to BrB9 VP7 was confirmed in P1 obtained in the 4th and 5th attempts (Fig. 5, Lane 4 and Lane 5).

VP4, which is common to a backbone virus WC3, was used as an internal control in performing RT-PCR. If BrB9 VP7 was reassorted into the WC3 virus, BrB9 VP7 and WC3 VP4 signals should be confirmed simultaneously from the sample, and WC VP7 should not be detected. Therefore, #4 and #5 successfully confirmed that BrB9 VP7 was reassorted into the WC3 virus.

### Example 8. Isolation of reassortant virus

As the viruses of the P0 and P1 stages exist as mixed clones rather than single clones, plaque isolation is performed using the P1 sample corresponding to Lane 4 of Fig. 5 to isolate the reassorted monoclonal virus. In order to visually observe the formed plaques, the cell monolayer was stained with neutral red to confirm the proliferation of the virus.

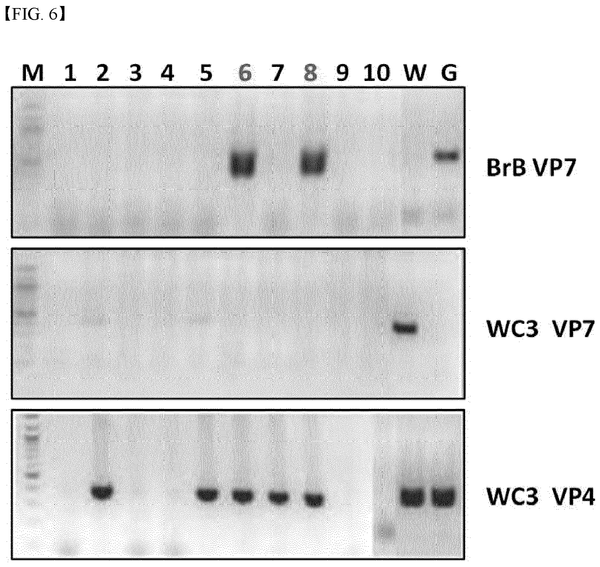

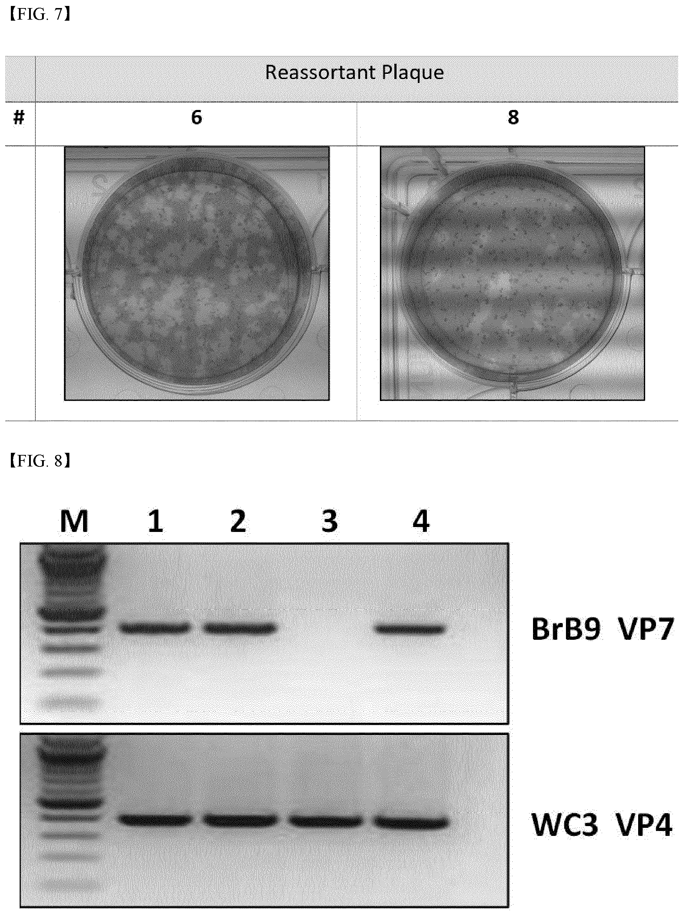

60 to 100 plaques were picked and analyzed, and the series of processes was continuously repeated three or more times to find reassorted plaques. The plaque picking process is as follows: 1) Mark candidates for reassorted plaques under a microscope or with the naked eye; 2) Dispense 300ul of EMEM (serum-free) into each e-tube according to the number to be picked; 3) Pick with a sterilized tip and place in 2); 4) Store at -70 °C or use for infection by adding trypsin (T0303, 1ug/mL). After the plaque assay was performed, 10 identified plaques were isolated. Plaque-isolated single clones were harvested after infection with the TK14r cell line (24well, 1 × 10E5/well) and cultured for 3 days or until CPE is confirmed. Viral RNA was extracted from the harvested sample and analyzed by RT-PCR using it as a template to determine whether it was a reassortant virus. As a result, clones #6 and #8 were confirmed as reassortant clones (Fig.6). Clones #6 and #8 identified as G4 reassortant plaques were cultured in the MA-104 cell line to obtain a reassortant virus stock (FIGs. 7 and 8).

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are intended to be embraced by the claims.

## Claims

1. A method for producing a reassortant rotavirus comprising:
providing a cell line stably expressing an inhibitory RNA against a specific gene segment among 11 gene segments of a first rotavirus;
introducing mRNA of a gene segment of a second rotavirus corresponding to said specific gene segment of the first rotavirus, or DNA or cDNA encoding the mRNA, into the cell line;
infecting the cell line with the first rotavirus; and
recovering a reassortant rotavirus comprising a gene segment of a second rotavirus corresponding to said specific gene segment of a first rotavirus.

2. The method of Claim 1, wherein the first rotavirus is a non-human rotavirus, bovine rotavirus, simian rotavirus, porcince rotavirus, canine rotavirus, or goat rotavirus.

3. The method of Claim 2, wherein the first rotavirus is a WC3 rotavirus strain or a progeny thereof.

4. The method of Claim 1, wherein the second rotavirus is a human rotavirus.

5. The method of Claim 4, wherein the second rotavirus is a P1, P2, G1, G2, G3, G4 or G9 human rotavirus serotype.

6. The method of Claim 1, wherein the second rotavirus is WI79, Wa, D, WISC2, DS-1, WI78, P, HCR3A, Br (Bricout) B, ST-3, BrB-9, WI79-9, SC2-9, or WI78-8 rotavirus strain, or a progeny thereof.

7. The method of Claim 1, wherein the specific gene segment is a gene segment encoding a protein selected from VP1, VP2, VP3, VP4, VP6, VP7, NSP1, NSP2, NSP3, NSP4, and NSP5.

8. The method of Claim 1, wherein the specific gene segment is a gene segment encoding VP7 or VP4.

9. The method of Claim 1, wherein the inhibitory RNA is miRNA, siRNA or shRNA.

10. The method of Claim 1, wherein the inhibitory RNA targets at least one selected from the group consisting of target sites of SEQ ID NO: 3, SEQ ID NO: 4, and SEQ ID NO: 5.

11. The method of Claim 10, wherein the inhibitory RNA is miRNA, and the cell line is introduced with a vector containing a double-stranded DNA oligonucleotide encoding a pre-miRNA for the miRNA.

12. The method of Claim 11, wherein the double-stranded DNA oligonucleotide is one or more double-stranded DNA oligonucleotides formed by one or more sequence pairs of selected from the group consisting of SEQ ID NO: 6 and SEQ ID NO: 7, SEQ ID NO: 8 and SEQ ID NO: 9, and SEQ ID NO: 10 and SEQ ID NO: 11.

13. The method of Claim 11, wherein the vector is a co-expression vector comprising two or more double-stranded DNA oligonucleotides encoding pre-miRNAs for two or more miRNAs having different target sites.

14. The method of Claim 1, wherein mRNA of the gene segment of the second rotavirus corresponding to the specific gene segment of the first rotavirus is obtained by in vitro transcription.

15. The method of Claim 1, after infecting the cell line with the first rotavirus, the method further comprises enriching the reassortant rotavirus through subculture.

16. The method of Claim 1, wherein the cell line is MRC-5, MA-104, Vero, BHK-21, COS-7, 293 T, CV-1 or TF-104 cell line.

17. The method of Claim 1, comprising:
providing a cell line stably expressing miRNA against a VP7 gene segment of bovine WC3 rotavirus;
introducing mRNA of a VP7 gene segment of human rotavirus, or DNA or cDNA encoding the mRNA into the cell line;
infecting the cell line with the WC3 rotavirus; and
recovering a reassorted rotavirus comprising the VP7 gene segment of human rotavirus.

18. An inhibitory RNA oligonucleotide that inhibits any one or more target sites selected from the group consisting of SEQ ID NO: 3, SEQ ID NO: 4, and SEQ ID NO: 5 for use in the method for producing a reassortant rotavirus according to Claim 1.

19. A double-stranded DNA oligonucleotide formed by any one or more sequence pairs selected from the group consisting of SEQ ID NO: 6 and SEQ ID NO: 7, SEQ ID NO: 8 and SEQ ID NO: 9, and SEQ ID NO: 10 and SEQ ID NO: 11 for use in the method for producing a reassortant rotavirus according to Claim 1.
